# EUROPEAN PATENT APPLICATION

(11) **EP 0 629 398 A1**
(43) Date of publication of application: **21.12.1994**
(21) Application number: 94304393.5
(22) Date of filing: 17.06.1994
(51) Int. Cl.: A61K 9/50

(54) **Pharmaceutical preparation for controlled release of a medicinal compound at a targeted site in the intestinal tract**

(30) Priority: 18.06.1993 JP 147954/93
(71) Applicant: TANABE SEIYAKU CO., LTD., Chuo-ku Osaka (JP)
(72) Inventor: Noda, Kazuo, Takarazuka-shi, Hyogo-ken (JP); Hirakawa, Yoshiyuki, Kobe-shi, Hyogo-ken (JP); Yoshino, Hiroyuki, Suita-shi, Osaka-fu (JP); Kubo nee Nagata, Minako, Takatsuki-shi, Osaka-fu (JP)
(74) Representative: Baverstock, Michael George Douglas

(57) **Abstract**

A pharmaceutical preparation controlled to release a medicinal compound at a targeted site in the intestinal tract comprises a medicinal compound-containing phase and an enteric coating phase provided around the medicinal compound-containing phase, said medicinal compound-containing phase releasing the medicinal compound quickly after a predetermined lag-time after the dissolution of the enteric coating phase. The pharmaceutical preparation makes it possible for the medicinal compound to be quickly released at any targeted site in the small intestine or the large intestine by the control of the amount of the enteric coating phase and the lag-time before the medicinal compound-containing phase starts to release the medicinal compound.

## Description

The present invention relates to a new pharmaceutical preparation controlled to release a medicinal compound at a targeted site in the intestinal tract, and more particularly to a pharmaceutical preparation from which a medicinal compound can be released selectively at a targeted site such as the large intestine by the control of the starting time of release of the medicinal compound.

Selective delivery of a medicinal compound to the large intestine is desired in some recent drug therapies, for instance, a local therapy for inflammatory disease in the large intestine such as ulcerative colitis, or an oral administrative therapy with a medicinal compound of a peptide which is easily decomposed chemically or enzymatically in the stomach or small intestine. In order to realize these therapies, various researches have been done and several methods for such therapies have been reported.

Among these methods are classical procedures such as methods using enteric pharmaceutical preparations and sustained release pharmaceutical preparations.

However, when using enteric pharmaceutical preparations, the release of a medicinal compound starts abruptly at the upper small intestine, resulting in consumption of almost all of the medicinal compound by absorption or decomposition before arrival of the medicinal compound at the large intestine, although release of the medicinal compound can be effectively suppressed in the stomach.

When using sustained release pharmaceutical preparations, a considerable amount of the medicinal compound is released when the pharmaceutical preparation stays in the stomach or passes through the small intestine before arrival of the medicinal compound at the large intestine, because the medicinal compound is continuously released.

Further, attempts have been made to suppress the release of a medicinal compound in the stomach by coating a sustained release pharmaceutical preparation with an enteric coat. However, the problem that the medicinal compound is released during the passage of the pharmaceutical preparation through the small intestine has not entirely been solved by the above-mentioned enteric-coated sustained release pharmaceutical preparation.

Moreover as a relatively new technique, it can be considered to utilize a technique of controlling the starting time of the release. However, taking into consideration that the residence time of a pharmaceutical preparation in the digestive tract is not always constant, it is difficult to envisage that this technique is suitable for the purpose of selective delivery of a medicinal compound to the large intestine.

From the viewpoint of the selectivity of the large intestine for releasing a medicinal compound, there has been recently developed a system utilizing the ecosystem of specific microorganisms in the large intestine. In such a system, there is used a pharmaceutical preparation wherein a composition containing a medicinal compound is coated with a new polymer having an azo group, or the composition containing a medicinal compound is dispersed in the new polymer having an azo group to form a matrix type of pharmaceutical preparation.

In the above-mentioned pharmaceutical preparation, the polymer is decomposed in the large intestine by enterobacteria having azo-reductase activity and the medicinal compound is thereby released. However, for practical use, there are still many problems to be solved, for example, regarding the safety of the polymer itself, and the controllability of its decomposition rate.

On the other hand, as to parenteral administration, hitherto rectal administration with a suppository or enema has often been employed to treat ulcerative colitis. However, in the case of the above-mentioned administration, there are problems due to the therapeutic method in that compliance to the method is low because of the inconvenience of administration, and the medicinal compound cannot be delivered to the upper large intestine such as the ascending colon or transverse colon.

An object of the present invention is to solve the above-mentioned problems in conventional pharmaceutical preparations, and to provide an orally administerable pharmaceutical preparation controlled to release a medicinal compound at a targeted site, by which a medicinal compound can be selectively delivered to a desired site of the small intestine or large intestine.

This and the other objects of the present invention will become apparent from the description hereinafter.

In accordance with the present invention, there is provided a pharmaceutical preparation controlled to release a medicinal compound at a targeted site in the intestinal tract, which comprises a medicinal compound-containing phase and an enteric coating phase provided around the medicinal compound-containing phase, said medicinal compound-containing phase releasing the medicinal compound quickly after a predetermined lag-time after the dissolution of the enteric coating phase.

The pharmaceutical preparation of the present invention controlled to release a medicinal compound at a targeted site makes it possible for a medicinal compound to be rapidly released at any targeted site in the small intestine or large intestine by the control of the amount of the enteric coating phase and/or the lag-time before the medicinal compound-containing phase starts to release the medicinal compound. Accordingly, any desired sites such as the duodenum, small intestine, colon and rectum can be targeted for delivery of the medicinal compound.

In the accompanying drawings:-

Fig. 1 shows a structure of an embodiment of the pharmaceutical preparation of the present invention which is controlled to release a medicinal compound at a targeted site.

Fig. 2 is a graph showing the result of the dissolution test with a first fluid and a second fluid (Japanese Pharmacopoeia) using a granule containing 5-aminosalicylic acid obtained in Example 1 which is controlled to release the medicinal compound at the large intestine.

Fig. 3 is a graph showing the result of the dissolution test with a second fluid using the granule containing 5-aminosalicylic acid obtained in Example 1 which is controlled to release the medicinal compound at the large intestine, after immersing the granule in the first fluid.

In order to efficiently realize the site-selective release of a medicinal compound, for example, at the large intestine, it is necessary to design a pharmaceutical preparation taking into account the physical and physiological environment in the human digestive tract and the residence time of the pharmaceutical preparation in the digestive tract. The value of pH in the stomach is usually recognized to be 1.8 to 4.5 in a healthy human. According to the report of D.F. Evans et al., the value of pH in the intestine is 6.5 to 7.5 and the pH does not substantially differ between the small intestine and the large intestine. According to the results of the widespread research of S.S. Davis et al., the residence time of a pharmaceutical preparation in the human stomach is 0.5 to 10 hours and further not only is the inter-individual variation large, but also the residence time is considerably influenced for example, by feeding and the size of the pharmaceutical preparation to be administered. That is, the intra-individual variation of the residence time is also large. On the other hand, the passage time of a pharmaceutical preparation through the small intestine is generally recognized to be 3±1 hours and the inter- and intra-individual variation is relatively small.

Based on the above-mentioned scientific findings, the pharmaceutical preparation of the present invention controlled to release a medicinal compound at a targeted site has been obtained by efficiently combining the technique of an enteric pharmaceutical preparation and the technique of a pharmaceutical preparation controlled to start the release of a medicinal compound after a predetermined lag-time.

The pharmaceutical preparation of the present invention controlled to release a medicinal compound at a targeted site comprises a the medicinal compound-containing phase which can rapidly release the medicinal compound after a predetermined lag-time (hereinafter, referred to as medicinal compound-containing phase) and an enteric coating phase thereon.

In the present invention, any film-formable high molecular substance soluble in aqueous solution at a pH of not less than 5 and insoluble in aqueous solution at a pH of less than 5 can be used as an enteric high molecular substance which forms the enteric coating phase of the pharmaceutical preparation of the present invention. Such high molecular substances include, for example, (1) a carboxyalkyl cellulose ether, (2) a cellulose derivative having a monoester bond of a dibasic acid, (3) a polyvinyl derivative having a monoester bond of a dibasic acid, (4) a maleic acid-vinyl compound copolymer, and (5) an acrylic copolymer.

Specific examples of the carboxyalkyl cellulose ether (1) include, for example, carboxymethylethyl-cellulose. Specific examples of the cellulose derivative (2) include, for example, cellulose acetate phthalate, cellulose acetate succinate, methylcellulose phthalate, hydroxymethylethylcellulose phthalate, hydroxypropylmethylcellulose phthalate, and hydroxypropylmethylcellulose acetate succinate. Specific examples of the polyvinyl derivative (3) include, for example, polyvinyl alcohol phthalate, polyvinyl butylate phthalate, and polyvinyl acetoacetal phthalate. Specific examples of the maleic acid-vinyl compound copolymer (4) include, for example, poly(vinyl acetate, maleic acid anhydride), poly(vinyl butyl ether, maleic acid anhydride), and poly(styrene, maleic acid monoester). Specific examples of the acrylic copolymer (5) include, for example, poly(methyl acrylate, methacrylic acid), poly(styrene, acrylic acid), poly(methyl acrylate, methacrylic acid, octyl acrylate), poly(methacrylic acid, methyl methacrylate) (e.g. Eudragit L and Eudragit S, each being trade names, available from Röhm Pharma, Germany), and poly(methacrylic acid, ethyl acrylate) (e.g. Eudragit L 30D-55, trade name, available from Röhm Pharma, Germany).

Among these examples, carboxymethylethylcellulose, hydroxypropylmethylcellulose acetate succinate and Eudragit L are preferably used as the enteric high molecular substance.

The above-mentioned high molecular substance can be used alone or, if necessary, in admixture with another enteric high molecular substance, a water-soluble high molecular substance or a water-insoluble high molecular substance.

As to the medicinal compound-containing phase in the present invention, there can be used any preparation which is controlled to start the release of the medicinal compound after a predetermined lag-time and quickly releases the medicinal compound once the release starts. The construction, the form of preparation, such as granules or tablets, the size, and the release mechanism of the medicinal compound-containing phase are not particularly limited.

The medicinal compound-containing phase in the present invention includes, for example, a pharmaceutical preparation which releases the medicinal compound in a sigmoid-type dissolution pattern or in a pulse-type dissolution pattern. Specific examples of the medicinal compound-containing phase in the present invention are, for example, (i) a controlled release pharmaceutical preparation wherein a core containing a medicinal compound and an organic acid is coated with a film formed by an aqueous coating of a water-insoluble and slightly water-permeable acrylic polymer having a trimethylammonioethyl group, (ii) a pharmaceutical preparation wherein a core containing a medicinal compound is coated with a film of a water-repellent substance and a water-insoluble and slightly water-permeable acrylic polymer having a trimethylammonioethyl group, and (iii) a pharmaceutical preparation wherein a core containing a medicinal compound and a water-swellable substance is coated, by means of compression coating, with a release-controlling layer comprising a water-soluble substance and a matrix-formable hydrophobic substance.

In the above-mentioned pharmaceutical preparation (i), any orally administerable medicinal compound can be used as the medicinal compound. As the organic acid, citric acid, succinic acid, fumaric acid, malic acid, and tartaric acid can suitably be used.

As the water-insoluble and slightly water-permeable acrylic polymer having a trimethylammonioethyl group in the pharmaceutical preparation (i), a polymer or copolymer of acrylic acid, methyl acrylate, ethyl acrylate, methacrylic acid, methyl methacrylate, or ethyl methacrylate, which has a trimethylammonioethyl group in the molecule, can be used. For instance, poly(ethyl acrylate, methyl methacrylate, trimethylammonioethyl methacrylate chloride) in which about 0.025 to about 0.033 mole of trimethylammonioethyl methacrylate chloride is contained per mole of the other neutral acrylic monomers is preferably used. Such a copolymer is, for example, Eudragit RS (trade name, available from Röhm Pharma, Germany).

The above-mentioned polymer may contain, for instance, a small quantity of a water-permeable polymer. Such a copolymer is, for example, Euragit RL (trade name, available from Röhm Pharma, Germany).

The pharmaceutical preparation (i) shows a sigmoid-type dissolution pattern wherein a release of the medicinal compound is restrained for a predetermined lag-time after the administration and once the dissolution starts, the medicinal compound dissolves quickly. The amount of the film is preferably about 0.3 to about 10 parts by weight, more preferably about 0.4 to about 5 parts by weight per part by weight of the organic acid. When the amount of the film is large, the lag-time of the pharmaceutical preparation becomes long, and when the amount of the organic acid is large, the dissolution rate of the medicinal compound becomes very rapid.
In the above-mentioned pharmaceutical preparation (ii), as the water-repellent substance, a salt of a higher fatty acid such as stearic acid and an alkaline earth metal is preferably used. Specific examples of the salts are calcium stearate, and magnesium stearate. As the water-insoluble and slightly water-permeable acrylic polymer having a trimethylammonioethyl group, the acrylic polymers exemplified in the above-mentioned pharmaceutical preparation (i) can be used.

The pharmaceutical preparation (ii) shows, like pharmaceutical preparation (i), a sigmoid-type dissolution pattern wherein release of the medicinal compound is restrained for a predetermined lag-time after the administration and once the dissolution starts, the medicinal compound dissolves quickly.

In the above-mentioned pharmaceutical preparation (iii), as the water-swellable substance, any substance which quickly swells with water can be used. Specific examples of the water-swellable substance are, for example, starch, cellulose, a carboxyalkylpolysaccharide such as carboxymethylcellulose or carboxymethylstarch, a hydroxyalkylpolysaccharide such as low-substituted hydroxypropylcellulose or hydroxypropylstarch, an alkali metal salt thereof such as the sodium salt, or an alkaline earth metal salt thereof such as the calcium salt.

The above-mentioned water-swellable substance is contained in the pharmaceutical preparation (iii) in an amount sufficient to burst the release-controlling layer by swelling with water, usually about 5 to about 30 w/w % based on the core, preferably.

As the water-soluble substance which constitutes the release-controlling layer in the pharmaceutical preparation (iii), a polyethyleneglycol having a molecular weight of 1500 to 20000, a saccharide such as sucrose, lactose, sorbitol or mannitol can be used. As the hydrophobic substance which constitutes the release-controlling layer, there can be used any hydrophobic substance which is matrix-formable by means of compression coating, for instance, a higher fatty acid ester, a higher hydrocarbon, or a mixture thereof.

Specific examples of the higher fatty acid ester are, for instance, an ester of a fatty acid having 10 to 32 carbon atoms and a fatty monohydric alcohol having 14 to 32 carbon atoms, (for instance, a fatty acid ester such as myristyl palmitate, stearyl stearate, myristyl myristate, ceryl lignocerate, lacceryl cerotate or lacceryl laccerate; a natural wax obtained from animals such as lanolin, beeswax, spermaceti or shellac wax; a natural wax obtained from plants such as carnauba wax or candelilla wax), an ester of a saturated or unsaturated fatty acid having 10 to 22 carbon atoms and glycerin or a hydrogenated compound thereof (for instance, glyceryl monolaurate, glyceryl monomyristate, glyceryl monostearate, glyceryl dilaurate, glyceryl dimyristate, glyceryl distearate, glyceryl trilaurate, glyceryl trimyristate, glyceryl tristearate, beef tallow, lard, hydrogenated beef tallow, hydrogenated rape seed oil, hydrogenated castor oil, hydrogenated coconut oil, and hydrogenated soybean oil). Specific examples of the higher hydrocarbons are, for instance, hydrocarbons having 12 to 32 carbon atoms (for instance, solid paraffin which is a mixture of various hydrocarbons).

In the release-controlling layer of the pharmaceutical preparation (iii), if necessary, a water-insoluble high molecular compound may be included in addition to the above-mentioned higher fatty acid ester or the above-mentioned higher hydrocarbon. Specific examples of the water-insoluble high molecular compound are, for example, ethylcellulose, polyvinyl chloride, poly(ethyl acrylate, methyl methacrylate), poly(ethyl acrylate, methyl methacrylate, and trimethylammonioethyl methacrylate chloride).

The above-mentioned release-controlling layer of the pharmaceutical preparation (iii) has a structure wherein the water-soluble substance is contained in a matrix made of the hydrophobic substance.

The thus obtained pharmaceutical preparation (iii) shows a pulse-type dissolution pattern wherein release of a medicinal compound is restrained for a predetermined lag-time after the administration and once the dissolution starts, the medicinal compound dissolves extremely quickly. The lag-time of the pharmaceutical preparation (iii) can be easily controlled by selecting each kind of water-soluble substance and hydrophobic substance, the mixing ratio and the amounts thereof.

The above-mentioned medicinal compound-containing phases are coated with the various above-mentioned enteric coating phases in the present invention so that the desired pharmaceutical preparation controlled to release the medicinal compound at a targeted site can be obtained. Preferable combinations of the medicinal compound-containing phase and the enteric coating phase are, combinations of the pharmaceutical preparation (i) and Eudragit L, the pharmaceutical preparation (ii) and Eudragit L, and the pharmaceutical preparation (iii) and Eudragit L. A particularly preferable combination is, as the medicinal compound-containing phase, the pharmaceutical preparation (i) wherein a core containing a medicinal compound and an organic acid is coated with a film of Eudragit RS by aqueous coating, and, as the enteric coating phase, Eudragit L.

The medicinal compound contained in the above-mentioned medicinal compound-containing phase in the present invention is not particularly limited as long as it is orally administerable. Specific examples of such medicinal compounds are vitamins, amino acids, peptides, chemotherapeutic agents, antibiotics, respiratory stimulants, antitussives, expectorants, anti-malignant tumor agents, autonomic agents, psychotropic agents, local anaesthetics, muscle relaxants, agents affecting the digestive organs, antihistamines, toxicopetic agents, hypnotics, sedatives, antiepileptics, antipyretics, analgesics, antiinflammatory agents, cardiotonics, antiarrhythmic agents, antihypertensives, diuretics, vasodilators, antilipemic agents, nutrients, tonics, alteratives, anticoagulants, agents for liver disease, hypoglycemics, and antihypertensives.

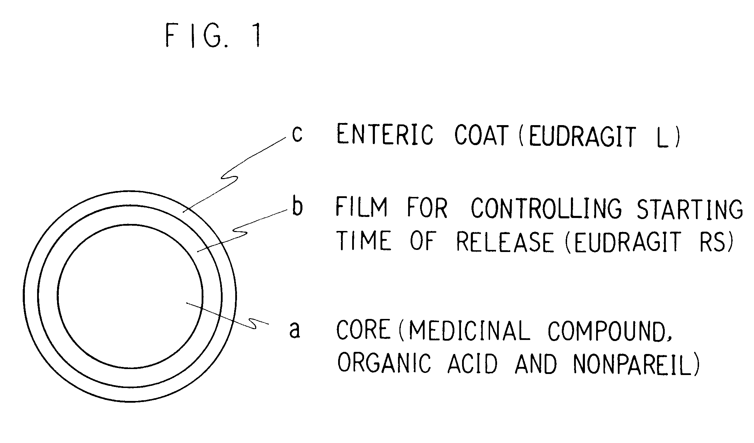

The present invention is more specifically described and explained by means of exemplifying a pharmaceutical preparation wherein the above-mentioned pharmaceutical preparation (i) is coated with a film of Eudragit L. The specific example of the pharmaceutical preparation of the present invention is a pharmaceutical preparation controlled to release a medicinal compound at the large intestine as shown in Fig. 1, wherein (a) a core containing a medicinal compound and an organic acid is coated with (b) a film for controlling the starting time of release formed by aqueous coating of a water-insoluble and slightly water-permeable acrylic polymer having a trimethylammonioethyl group, in this case, Eudragit RS, and further coated thereon with (c) an enteric coat made of an enteric high molecular substance, in this case, Eudragit L. The preparation composed of the core (a) and the film (b) corresponds to the medicinal compound-containing phase in the present invention and the enteric coat (c) corresponds to the enteric coating phase in the present invention.

In the above-mentioned pharmaceutical preparation controlled to release a medicinal compound at the large intestine, the function of the enteric coat, Eudragit L, is to prevent the penetration of water during the residence time of the pharmaceutical preparation in the stomach so that the medicinal compound-containing phase comprising the core (a) and the film (b) can be protected, and to dissolve quickly with the increase in the surrounding pH after discharge of the pharmaceutical preparation from the stomach so that the medicinal compound-containing phase is passed to the upper small intestine. Then, the medicinal compound-containing phase passes through the small intestine without releasing the medicinal compound at all owing to the effect of the film (b) in controlling the starting time of the release of the medicinal compound. Further, when the medicinal compound-containing phase reaches the targeted upper large intestine, the organic acid which is gradually dissolved by liquid penetrating through the film (b), interacts with the film (b), for instance, Eudragit RS, to change abruptly its penetratability, resulting in start of the release of the medicinal compound from the medicinal compound-containing phase.

Thus the pharmaceutical preparation of the present invention shows a unique property in releasing the medicinal compound.

The final form of such an embodiment of the present invention is a granule having a particle diameter of 300 to 3000 µm, which is suitably divided into a unit pack or filled into capsules, respectively in a proper amount to be used for treatment.

As to the above-mentioned embodiment, in order to sufficiently exhibit the essential characteristic of the pharmaceutical preparation of the present invention, it is desirable to determine the amount of the enteric coat (c) so that the pharmaceutical preparation has sufficient acid resistance for a period of at least 6 hours which is recognized as the maximum residence time of the pharmaceutical preparation in the stomach, and to determine the amount of the film (b) so that after the dissolution of the enteric coat (c) the lag-time before the start of the release of the medicinal compound is 3±1 hours which is an average passage time for the pharmaceutical preparation through the small intestine.

In the above-mentioned embodiment which satisfies the above-mentioned requirements, the amount of the enteric coat (c) is 3 to 100 w/w % based on a total weight of the core (a) and the film (b), and the amount of the film (b) is 5 to 80 w/w % based on the weight of the core (a).

In the above-mentioned embodiment, by changing the kind and the amount of the enteric coat (c) and/or by changing the amount of the film (b) to control the lag-time for releasing the medicinal compound, a desired site in the small intestine or large intestine can be targeted for releasing the medicinal compound. If the targeted site is the small intestine, the amount of the enteric coat (c) is 3 to 100 w/w % based on a total weight of the core (a) and the film (b), and the amount of the film (b) is 5 to 40 w/w % based on a weight of the core (a).

The site-targeting method for releasing a medicinal compound is not limited to the method in the above-mentioned embodiment, and any lag-time controlling method suitably selected according to the medicinal compound-containing phase can be properly used in the present invention.

As described above, the pharmaceutical preparation of the present invention which is controlled to release a medicinal compound at a targeted site exhibits the effect that the medicinal compound is not released not only during a residence time in stomach, but also during a passage time through the small intestine (2 to 4 hours or more after the discharge from the stomach), and the eventual release of the medicinal compound starts quickly at a targeted site.

The pharmaceutical preparation of the present invention can be prepared by coating a medicinal compound-containing phase with an enteric coating phase (enteric coat).

The medicinal compound-containing phase can be prepared according to methods generally used in the art of pharmaceutical preparation. For instance, the pharmaceutical preparation (i) may be prepared by coating the core in a form such as plain tablet, granule, pill or fine granule containing a medicinal compound and an organic acid, with an aqueous dispersion of a water-insoluble and slightly water-permeable acrylic polymer having a trimethylammonioethyl group.

The preparation of the core can be carried out according to the usual preparative procedures, for example, as described in Lemingtons Pharmaceutical Sciences, 17, 1603-1632, 1633-1643 (Mack Publishing Company, published in 1985). For example, the core can be prepared by firstly mixing a medicinal compound, an organic acid and, if necessary, other additives such as an excipient, a binder and a lubricant, and secondly by preparing the above-mentioned form of preparation by the method of wet oscillating granulation, dry granulation, centrifugal fluidizing type coating, pan coating, or fluidized bed coating.

Instead of mixing the medicinal compound and an organic acid, the core can be prepared by forming a layer of the organic acid around an inert carrier particle and then by forming a layer of the medicinal compound thereon by adhesion of the medicinal compound, or alternatively, by forming a layer of the medicinal compound around the inert carrier particle and then by forming a layer of the organic acid thereon by adhesion of the organic acid, respectively giving a two-layer structure.

Alternatively, the core can be prepared by forming a layer of the organic acid around the carrier particle by adhesion of the organic acid, then by forming a film of a water-insoluble substance, for instance, ethylcellulose, or a wax such as a hydrogenated oil, thereon, which film controls dissolution of the organic acid, and by further forming a layer of the medicinal compound thereon by adhesion of the medicinal compound.

As the above-mentioned inert carrier particles, carrier particles made of sucrose, lactose, starch, or crystalline cellulose can suitably be used.

In the above-mentioned pharmaceutical preparation (i), the coating can be carried out by spraying an aqueous dispersion of an acrylic polymer on the core followed by drying, whereby the acrylic polymer adheres around the core.

As the coating method, a method generally used in the art of pharmaceutical preparation such as fluidized bed coating or pan coating can be employed. For example, in the case of the method of fluidized bed coating, the coating can be carried out as follows. That is, while the cores are fluidized in an apparatus by means of air pressure, the cores are spray-coated with an aqueous dispersion of an acrylic polymer at an adequate rate from the nozzle of the spray-gun.

The concentration of an acrylic polymer in the aqueous dispersion is not particularly limited, but preferably about 5 to about 40 w/w %.

In addition, a plasticizer or a coloring agent may be included in the aqueous dispersion. As a plasticizer, for instance, triacetin, triethyl citrate, acetyltributyl citrate, diethyl phthalate, polyethyleneglycol, or polysorbate can be suitably used. The amount of plasticizer to be used is preferably about 5 to about 40 w/w % based on the weight of the acrylic polymer.

Moreover, to the aqueous dispersion of an acrylic polymer, a hydrophilic organic solvent can be added in small quantity, and further, if necessary, talc, or titanium dioxide can be added as an aggregation-preventing agent.

Drying of the film of the acrylic polymer of the pharmaceutical preparation (i) can be easily carried out, for example, by heating at about 35⁰C to about 100⁰C, preferably about 40⁰C to about 70⁰C.

The pharmaceutical preparation (ii) can be prepared by coating a core containing a medicinal compound with a dispersion of a water-insoluble and slightly water-permeable acrylic polymer and a water repellent substance, followed by drying to give a film coated product. The preparation of the above-mentioned core and the coating of the core are suitably carried out according to the same method as exemplified for the above-mentioned pharmaceutical preparation (i).

The pharmaceutical preparation (iii) can be prepared by coating a core containing a medicinal compound and a water-swellable substance with a mixture of a water-soluble substance and a hydrophobic substance by means of compression coating.

The core of the pharmaceutical preparation (iii) can be prepared by methods generally used in the art of pharmaceutical preparation, for instance, by mixing a medicinal compound, a water-swellable substance and, if necessary, other additives such as excipients, binders and lubricants, and by tabletting the mixture obtained as it is or granules prepared from the mixture.

The compression coating of the core may be carried out according to a conventional method by means of a tabletting machine.

The mixture of a water-soluble substance and a hydrophobic substance to be coated around the core by compression coating can be used as it is or in the form of a granule prepared from the mixture. Such a granule used for tabletting can be prepared by mixing the water-soluble substance and the hydrophobic substance uniformly, and then by granulating the mixture obtained, followed by sieving, according to a conventional method. A uniform mixture of a water-soluble substance and a hydrophobic substance can be easily prepared as follows, by using a water-soluble substance which melts at such a temperature such that the hydrophobic substance to be used with it does not melt. For instance, polyethyleneglycol 6000 may be used as a water-soluble substance when hydrogenated castor oil is used as the hydrophobic substance. The above-mentioned uniform mixture of such substances can be easily prepared by mixing both substances in a state wherein only the water-soluble substance is molten.

The compression coating of the pharmaceutical preparation (iii) is preferably carried out under conditions such that the compressing pressure is, for instance, 200 to 1200 kg/cm² and the compressing rate is 1 to 20 mm/minute, preferably 5 to 10 mm/minute.

In addition, in the above-mentioned pharmaceutical preparations (i) to (iii), various additives such as excipients, binders, lubricants and aggregation-preventing agents which are generally used in this field may be included.

Specific examples of the excipient are a sugar such as sucrose, lactose, mannitol or glucose, starch, crystalline cellulose, calcium phosphate, and calcium sulfate. Specific examples of the binder are polyvinylalcohol, polyacrylic acid, polymethacrylic acid, polyvinylpyrrolidone, glucose, sucrose, lactose, maltose, dextrin, sorbitol, mannitol, hydroxyethylcellulose, hydroxypropylmethylcellulose, hydroxypropylcellulose, a macrogol, arabic gum, gelatin, agar, and starch. Specific examples of the lubricant and the aggregation-preventing agent are talc, magnesium stearate, calcium stearate, colloidal silica, stearic acid, waxes, hydrogenated oils, polyethyleneglycols, and sodium benzoate.

In the present invention, the method for coating the medicinal compound-containing phase with an enteric coating phase is not particularly limited, and both aqueous coating methods and non-aqueous coating methods generally used in this field can be employed. For instance, the coating can be carried out according to methods generally used in the art of preparation such as methods of spray coating or methods of fusion coating, in fluidized bed coating processes, tumbling and fluidized type coating processes, or pan coating processes.

In addition, in the enteric coating phase of the pharmaceutical preparation of the present invention, a plasticizer, or an aggregation-preventing agent can be included as in the general coat. As a plasticizer, for instance, triacetin, triethyl citrate, acetyltributyl citrate, diethyl phthalate, polyethyleneglycol, or polysorbate can be suitably used. The amount of the plasticizer to be used is preferably about 5 to about 40 w/w % based on the weight of the enteric high molecular substance.

Although the amount of the enteric coating phase varies according to the kind of medicinal compound, the kind of enteric high molecular substance and the targeted site for release, it can be suitably determined in each case. Generally, the amount of the enteric coating phase is preferably about 3 to about 100 w/w %, more preferably 5 to 5 0 w/w %, based on the medicinal compound-containing phase.

The present invention is more specifically described and explained by means of the following Examples. It is to be understood that the present invention is not limited to the Examples, and various changes and modifications may be made in the invention without departing from the scope thereof.

### Example 1

Nonpareil (spherically granulated sucrose, commercial name, from Freund Industrial Co. Ltd., Japan) having a particle diameter of 475 to 655 µm (1500 g) was put into a centrifugal fluidizing type coating and granulating apparatus (CF-360EX Type, made by Freund Industrial Co. Ltd., Japan, hereinafter, referred to as CF apparatus) and rolled in it. Thereto was gradually added a mixture of fine powder of 5-aminosalicylic acid (900 g) and succinic acid (1500 g) while spraying a solution of sucrose (660 g) dissolved in a mixture of water and ethanol (3:1, w/w, hereinafter the same) (1980 g) to coat the Nonpareil. A core containing 5-aminosalicylic acid in the form of a plain granule was thus prepared.

Then the prepared core (150 g) containing 5-aminosalicylic acid was put into a CF apparatus and spray-coated with a coating solution of Eudragit RS PM (trade name, available from Röhm Pharma, Germany) (41.85 g), talc (13.95 g) and Myvacet 9-45 (commercial name, available from Eastman Chemicals Co. Ltd., USA, of which generic name is distilled monoacetylglyceride (4.2 g) dissolved in a mixture of water and ethanol (2:1) (240 g) while blowing hot air at 50°C. After coating, the granule was dried by heating at 60°C to obtain a 5-aminosalicylic acid-containing phase in a form of granules which is controlled to start the release of the medicinal compound after a predetermined lag-time (yield: 210 g).

Then, the prepared 5-aminosalicylic acid-containing phase (170 g) was put into a CF apparatus and spray-coated with a coating solution consisting of Eudragit L30D-55 (trade name, available from Röhm Pharma, Germany), (195.35 g), talc (17.58 g), polyethyleneglycol 6000 (5.88 g), Tween 80 (2.94 g) and water (390.70 g) while blowing hot air at 60°C to obtain a pharmaceutical preparation of the present invention containing 5-aminosalicylic acid in a form of granules which is controlled to release the medicinal compound at the large intestine (yield: 210 g).

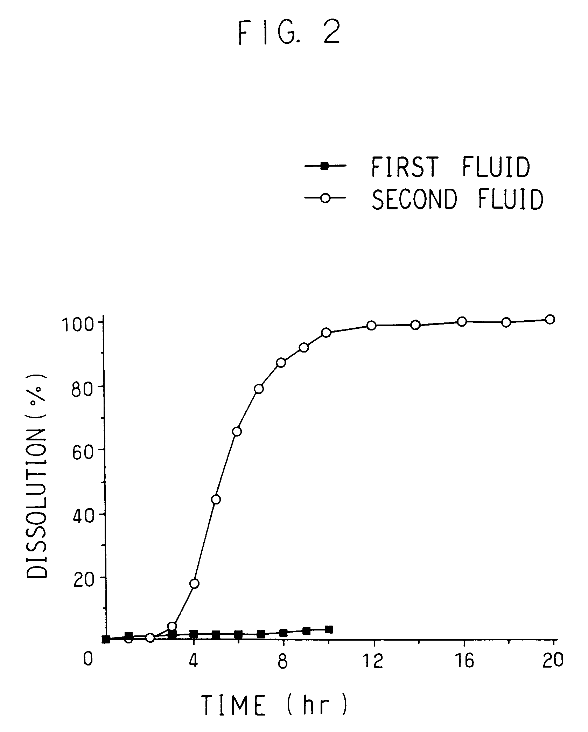

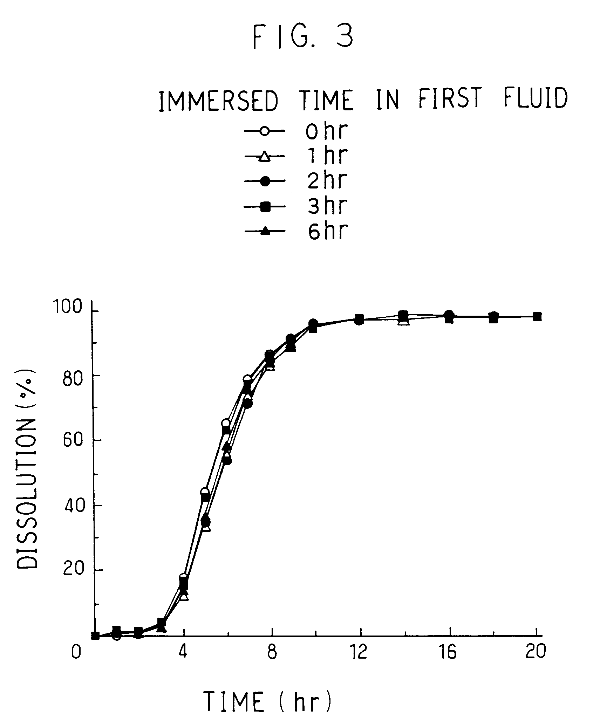

In order to confirm the effect of the pharmaceutical preparation of the present invention, with respect to the pharmaceutical preparation containing 5-aminosalicylic acid obtained as above which is controlled to release the medicinal compound at the large intestine, the following various dissolution tests were carried out with a first fluid of JPXII (pH 1.2) and with a second fluid of JPXII (pH 6.8) according to the puddle method (37°C, 100 rpm) based on the specification of the dissolution test in accord with JPXII.
(i) With respect to the pharmaceutical preparation obtained as above, the dissolution test was carried out with the first fluid or with the second fluid. The result is shown in Fig. 2 comparing each dissolution pattern with the other. In the first fluid, the medicinal compound was not released even after 10 hours, and from that it is seen that acid resistance of the pharmaceutical preparation was maintained sufficiently. In the second fluid, the medicinal compound was quickly released after a lag-time of about 3.5 hours.
   The reason why the pharmaceutical preparation obtained as above shows such a unique dissolution pattern in the second fluid is explained as follows. At first, the enteric coating phase which is the outer layer of the pharmaceutical preparation is dissolved, successively the dissolution medium penetrates into the medicinal compound-containing phase and gradually dissolves succinic acid, and then the dissolved succinic acid interacts with the film of Eudragit RS to change the penetrability thereof abruptly. Thus the release of the medicinal compound is restrained until the change in the penetrability of the film.
(ii) With respect to the pharmaceutical preparation obtained as above, after previous immersion in the first fluid for a certain time, the dissolution test was carried out with the second fluid. The result is shown in Fig. 3. Independent of the immersion time in the first fluid, in the second fluid each pharmaceutical preparation shows almost the same dissolution pattern of which the lag-time is about 3.5 hours.

The above-described results suggest that when the pharmaceutical preparation of the present invention is orally administered, without being affected by the variation of the residence time of the pharmaceutical preparation in the stomach, the eventual release of the medicinal compound starts at about 3.5 hours after passing to the small intestine, namely on arrival at the upper large intestine without any consumption of the medicinal compound before that time.

### Example 2

Nonpareil having a particle diameter of 475 to 655 µm (180 g) was put into a Wurster type granulating and coating apparatus (Glatt GPCG1, made by Glatt GmbH Process Technology, Germany) and fluidized in it, and then spray-coated with a solution of 5-aminosalicylic acid (24.0 g), hydroxypropylcellulose-SL (2.4 g) and polyvinylpyrrolidone-3 OK (2.4 g) suspended in a mixture of water and ethanol (1:95) (121.2 g) to obtain plain granules containing 5-aminosalicylic acid. Then the plain granules obtained were put into a CF apparatus and rolled in it. Thereto was gradually added a fine powder of succinic acid (40 g) while spraying a solution of sucrose (120 g) dissolved in a mixture of water and ethanol (3:1) (360 g). A core wherein Nonpareil was coated with 5-aminosalicylic acid and succinic acid in a form of granules was thus prepared.

Then the core (200 g) obtained was put into a CF apparatus, rolled in it, and spray-coated with a coating solution consisting of Eudragit RS 30D (trade name, available from Röhm Pharma, Germany) (210.24 g), talc (30.73 g), triethyl citrate (6.20 g) and water (291.91 g) while blowing hot air at 50°C. After coating, the granule was dried by heating at 60°C to obtain a 5-aminosalicylic acid-containing phase in a form of granule which is controlled to start the release of the medicinal compound after a predetermined lag-time (yield: 300 g).

Then, the 5-aminosalicylic acid-containing phase (100 g) obtained was put into a CF apparatus and spray-coated with a coating solution consisting of Eudragit L30D-55 (57.46 g), talc (5.17 g), polyethyleneglycol 6000 (1.73 g), Tween 80 (0.86 g) and water (114.91 g) while blowing hot air at 40°C to obtain a pharmaceutical preparation of the present invention containing 5-aminosalicylic acid in the form of granule which is controlled to release the medicinal compound at the large intestine (yield: 125 g).

### Example 3

Nonpareil having a particle diameter of 475 to 655 µm (750 g) was put into a CF apparatus and rolled in it. Thereto was gradually added a mixture of fine powder of lysozyme chloride (450 g) and succinic acid (450 g) while spraying a solution of sucrose (247 g) dissolved in a mixture of water and ethanol (3: 1) (740 g) to coat the Nonpareil. Plain granule containing lysozyme chloride was thus prepared. Then the plain granule obtained was put into a CF apparatus and rolled in it. Thereto was gradually added a fine powder of succinic acid (450 g) while spraying a solution of sucrose (123 g) dissolved in a mixture of water and ethanol (3:1) (493 g). A core coated with lysozyme chloride and succinic acid in a form of granule was thus prepared.

Then the core obtained (150 g) was put into a CF apparatus and spray-coated with a coating solution of Eudragit RS PM (19.58 g), talc (19.58 g) and Myvacet 9-45 (5.85 g) dissolved in a mixture of water and ethanol (2:1) (180 g) while blowing hot air at 50°C to obtain a lysozyme chloride-containing phase in a form of granule which is controlled to start the release of the medicinal compound after a predetermined lag-time (yield: 195 g).

Then, the lysozyme chloride-containing phase obtained (150 g) was put into a CF apparatus and spray-coated with a coating solution consisting of Eudragit L3 OD-55 (138.00 g), talc (12.41 g), polyethyleneglycol 6000 (4.15 g), Tween 80 (2.07 g) and water (275.79 g) while blowing hot air at 50°C to obtain a pharmaceutical preparation of the present invention containing lysozyme chloride in the form of granule which is controlled to release the medicinal compound at the large intestine (yield: 210 g).

### Example 4

Crystals of acetaminophen having a particle diameter of 475 to 655 µm (1050 g) was put into a CF apparatus and rolled in it. Thereto was gradually added a fine powder of acetaminophen (262.5 g) while spraying a solution of hydroxypropylcellulose-SL (6.5 g) dissolved in a mixture of water and ethanol (7:3) (650 g) to coat the crystals. Plain granule containing acetaminophen was thus prepared. Then the plain granule obtained was put into a CF apparatus and rolled in it. Thereto was gradually added a mixed fine powder of succinic acid (1050 g), sucrose (367.5 g) and hydrated silica (2.63 g) while spraying a solution of hydroxypropylcellulose-SL (30 g) dissolved in a mixture of water and ethanol (7:3) (970 g). The core wherein acetaminophen was coated with succinic acid in the form of granule was thus prepared.

Then the core obtained (250 g) was put into a CF apparatus and spray-coated with a coating solution consisting of Eudragit RS 30D (280.98 g), talc (41.07 g), triethyl citrate (8.29 g) and water (390.1 g) while blowing hot air at 50°C. After coating, the granule was dried by heating at 60°C to obtain an acetaminophen-containing phase in a form of granule which is controlled to start the release of the medicinal compound after a predetermined lag-time (yield: 350 g).

Then, the acetaminophen-containing phase obtained (270 g) was put into a CF apparatus and spray-coated with a coating solution consisting of hydroxypropylmethylcellulose acetate succinate-MF (30.34 g), stearic acid (15.17 g), triethyl citrate (8.49 g) and water (552.74 g) while blowing hot air at 50°C. After coating, the granule was dried by heating at 60⁰C to obtain a pharmaceutical preparation of the present invention containing acetaminophen in a form of granule which is controlled to release the medicinal compound at the large intestine (yield: 324 g).

### Example 5

Nonpareil having a particle diameter of 655 to 780 µm (1250 g) was put into a CF apparatus and rolled in it. Thereto was gradually added a mixture of fine powder of theophylline (750 g) and succinic acid (1250 g) while spraying a solution of sucrose (400 g) dissolved in a mixture of water and ethanol (3:1) (1200 g) to coat the Nonpareil. A core containing theophylline in a form of plain granule was thus prepared.

Then the core obtained (150 g) containing theophylline was put into a CF apparatus and spray-coated with a coating solution consisting of Eudragit RS 30D (94.61 g), talc (13.83 g), triethyl citrate (2.79 g) and water (131.36 g) while blowing hot air at 50°C. After coating, the granule was dried by heating at 60°C to obtain a theophylline-containing phase in a form of granule which is controlled to start the release of the medicinal compound after a predetermined lag-time (yield: 195 g).

Then, the theophylline-containing phase (150 g) obtained was put into a CF apparatus and spray-coated with a coating solution consisting of Eudragit L30D-55 (103.42 g), talc (9.3 g), polyethyleneglycol 6000 (3.11 g), Tween 80 (1.56 g) and water (206.85 g) while blowing hot air at 50°C to obtain a pharmaceutical preparation of the present invention containing theophylline in a form of granule which is controlled to release the medicinal compound at the large intestine (yield: 195 g).

### Example 6

Preparation of a pharmaceutical preparation for which the targeting site for release is the lower small intestine:

Nonpareil having a particle diameter of 655 to 780 µm (1250 g) was put into a CF apparatus and rolled in it. Thereto was gradually added a mixture of a fine powder of theophylline (750 g) and succinic acid (1250 g) while spraying a solution of sucrose (400 g) dissolved in a mixture of water and ethanol (3:1) (1200 g) to coat the Nonpareil. A core containing theophylline in a form of plain granule was thus prepared.

Then the core obtained (150 g) containing theophylline was put into a CF apparatus and spray-coated with a coating solution consisting of Eudragit RS 30D (31.54 g), talc (4.61 g), triethyl citrate (0.93 g) and water (43.79 g) while blowing hot air at 50°C. After coating, the granule was dried by heating at 60°C to obtain a theophylline-containing phase in the form of granule which is controlled to start the release of the medicinal compound after a predetermined lag-time (yield: 165 g).

Then, the theophylline-containing phase obtained (150 g) was put into a CF apparatus and spray-coated with a coating solution consisting of Eudragit L30D-55 (103.42 g), talc (9.3 g), polyethyleneglycol 6000 (3.11 g), Tween 80 (1.56 g) and water (206.85 g) while blowing hot air at 50°C to obtain a pharmaceutical preparation of the present invention containing theophylline in a form of granule which is controlled to release the medicinal compound at the lower small intestine (yield: 195 g).

In addition to the ingredients used in the Examples, other ingredients can be used in the Examples as set forth in the specification to obtain substantially the same results.

## Claims

1. A pharmaceutical preparation controlled to release a medicinal compound at a targeted site in the intestinal tract, which comprises a medicinal compound-containing phase and an enteric coating phase provided around the medicinal compound-containing phase, said medicinal compound-containing phase releasing the medicinal compound quickly after a predetermined lag-time after the dissolution of the enteric coating phase.

2. A pharmaceutical preparation as claimed in Claim 1 wherein the medicinal compound-containing phase releases the medicinal compound in a sigmoid-type dissolution pattern or in a pulse-type dissolution pattern.

3. A pharmaceutical preparation as claimed in Claim 1 or Claim 2, wherein the medicinal compound-containing phase has a lag-time such that the medicinal compound is released at the large intestine.

4. A pharmaceutical preparation as claimed in any one of the preceding claims, wherein the medicinal compound-containing phase is a preparation comprising (1) a core containing a medicinal compound and an organic acid, and (2) a film coat formed around the core by aqueous coating of a water-insoluble and slightly water-permeable acrylic polymer having a trimethylammonioethyl group.

5. A pharmaceutical preparation as claimed in any one of Claims 1 to 3, wherein the medicinal compound-containing phase is a preparation comprising (1) a core containing a medicinal compound, and (2) a film coat provided around the core containing a water-repellent substance and a water-insoluble and slightly water-permeable acrylic polymer having a trimethylammonioethyl group.

6. A pharmaceutical preparation as claimed in any one of Claims 1 to 3, wherein the medicinal compound-containing phase is a preparation comprising (1) a core containing a medicinal compound and a water-swellable substance, and (2) a coat formed around the core by compression coating, said coat containing a water-soluble substance and a matrix-formable hydrophobic substance.

7. A pharmaceutical preparation as claimed in any one of the preceding claims, wherein the enteric coating phase is a film coat containing an enteric high molecular substance.

8. A pharmaceutical preparation as claimed in Claim 4 or Claim 5, wherein the water-insoluble and slightly water-permeable acrylic polymer having a trimethylammonioethyl group is poly(ethyl acrylate, methyl methacrylate, trimethylammonioethyl methacrylate chloride).

9. A pharmaceutical preparation as claimed in Claim 4, wherein the organic acid is succinic acid.

10. A pharmaceutical preparation as claimed in Claim 7, wherein the enteric high molecular substance is an acrylic copolymer or a cellulose derivative.
